# EUROPEAN PATENT APPLICATION

(11) **EP 4 523 732 A1**
(43) Date of publication of application: **19.03.2025**
(21) Application number: 23803431.8
(22) Date of filing: 26.04.2023
(51) Int. Cl.: A61N 1/36

(54) **BEAUTY DEVICE AND METHOD FOR CONTROLLING SAME**

(30) Priority: 12.05.2022 JP 2022078874
(71) Applicant: Panasonic Intellectual Property Management Co., Ltd., Kadoma-shi, Osaka 571-0057 (JP)
(72) Inventor: HOSHIBA Taichi, Kadoma-shi, Osaka 571-0057 (JP); IIMURA Ruriko, Kadoma-shi, Osaka 571-0057 (JP); KANEKO Shota, Kadoma-shi, Osaka 571-0057 (JP)
(74) Representative: Grünecker Patent- und Rechtsanwälte PartG mbB
(86) International application number: PCT/JP2023/016382
(87) International publication number: WO 2023/218952

(57) **Abstract**

Beauty device (1A) includes electrode group (22) having three or more electrodes (23) through which a current flows in a skin of a user. Further, beauty device (1A) includes control unit (13a) that changes a setting at predetermined time intervals while setting two or more positive electrodes and one or more negative electrodes in electrode (23) included in electrode group (22). Control unit (13a) makes characteristics of the current applied by each of the first positive electrode and the second positive electrode, which are selected from two or more positive electrodes, different from each other.

## Description

### TECHNICAL FIELD

The present disclosure relates to a beauty device and a method for controlling the beauty device.

### BACKGROUND ART

Conventionally, there is a beauty device that gives a beauty effect to a skin by energizing a skin surface of a user in a state where an electrode is in contact with the skin surface. For example, Patent Literature 1 discloses a technique related to a beauty device that includes a plurality of electrodes arranged along a circumferential direction and energizes a predetermined combination of electrodes. In this beauty device, a region on the skin to be energized changes with time by changing a combination of electrodes.

### Citation List

### Patent Literature

PTL 1: Unexamined Japanese Patent Publication No. 2020-185207

### SUMMARY OF THE INVENTION

The beauty device disclosed in Patent Literature 1 can give a stimulus to the skin only between the electrodes according to a predetermined combination, and thus has poor sense of use for the skin.

The present disclosure provides a beauty device and a method for controlling the beauty device that give a stimulus that provides a user's skin with more sense of use.

A beauty device according to a first aspect of the present disclosure includes: an electrode group including three or more electrodes through which a current flows in a skin of a user; and a control unit that performs a setting of selecting two or more positive electrodes and one or more negative electrodes from the electrodes included in the electrode group, the setting is changed at predetermined time intervals. The control unit makes characteristics of the current applied by each of a first positive electrode and a second positive electrode, which are selected from the two or more positive electrodes, different from each other.

A method for controlling a beauty device according to a second aspect of the present disclosure is a method for controlling a beauty device including an electrode group having three or more electrodes through which a current flows in a skin of a user. The method for controlling a beauty device includes a step of changing a setting at predetermined time intervals while setting two or more positive electrodes and one or more negative electrodes for the electrodes included in the electrode group. In the step, characteristics of the current applied by each of a first positive electrode and a second positive electrode selected from the two or more positive electrodes are different from each other.

According to the present disclosure, it is possible to provide the beauty device and the method for controlling the beauty device that give a stimulus that provides a user's skin with more sense of use.

### BRIEF DESCRIPTION OF THE DRAWINGS

Fig. 1 is a perspective view of a beauty device according to a first exemplary embodiment.
Fig. 2 is an enlarged view of a head part provided in the beauty device according to the first exemplary embodiment.
Fig. 3A is a view illustrating a first stage of a first use mode in the first exemplary embodiment.
Fig. 3B is a view illustrating a second stage of the first use mode in the first exemplary embodiment.
Fig. 3C is a view illustrating a third stage of the first use mode in the first exemplary embodiment.
Fig. 3D is a view illustrating a fourth stage of the first use mode in the first exemplary embodiment.
Fig. 4 is a perspective view illustrating the beauty device to which an extended structure is connected.
Fig. 5 is a partially enlarged view of the extended structure.
Fig. 6 is a perspective view of a beauty device according to a second exemplary embodiment.
Fig. 7 is a plan view of the beauty device according to the second exemplary embodiment.
Fig. 8 is an exploded view of the beauty device according to the second exemplary embodiment.
Fig. 9 is a cross-sectional view of a mask main body corresponding to a cross section taken along line IX-IX in Fig. 7.
Fig. 10A is a view illustrating a first stage of a first use mode in the second exemplary embodiment.
Fig. 10B is a view illustrating a second stage of the first use mode in the second exemplary embodiment.
Fig. 10C is a view illustrating a third stage of the first use mode in the second exemplary embodiment.
Fig. 10D is a view illustrating a fourth stage of the first use mode in the second exemplary embodiment.
Fig. 11A is a view illustrating a first stage of a second use mode in the second exemplary embodiment.
Fig. 11B is a view illustrating a second stage of the second use mode in the second exemplary embodiment.
Fig. 11C is a view illustrating a third stage of the second use mode in the second exemplary embodiment.
Fig. 11D is a view illustrating a fourth stage of the second use mode in the second exemplary embodiment.

### DESCRIPTION OF EMBODIMENT

Hereinafter, exemplary embodiments will be described in detail with reference to the drawings. However, unnecessarily detailed description may be omitted. For example, a detailed description of a well-known matter or a repeated description of substantially the same configuration may be omitted. The accompanying drawings and the following description are provided for those skilled in the art to fully understand the present disclosure, and are not intended to limit the subject matter described in the claims.

### (First exemplary embodiment)

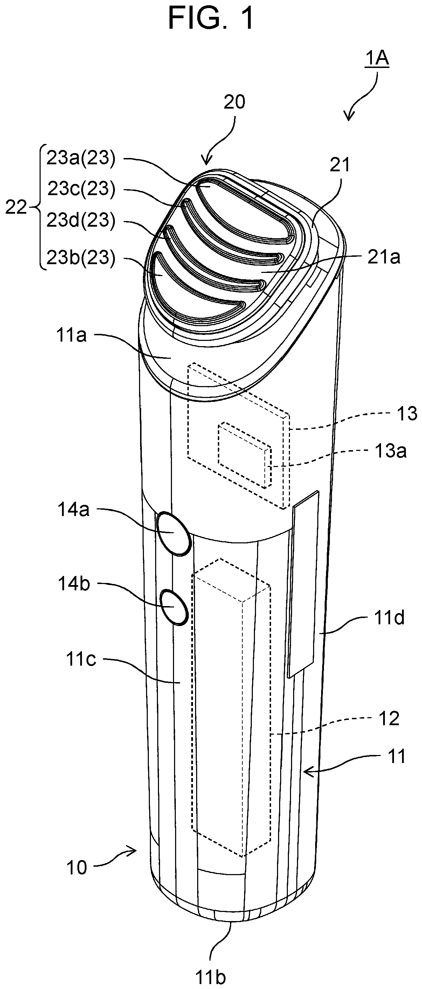

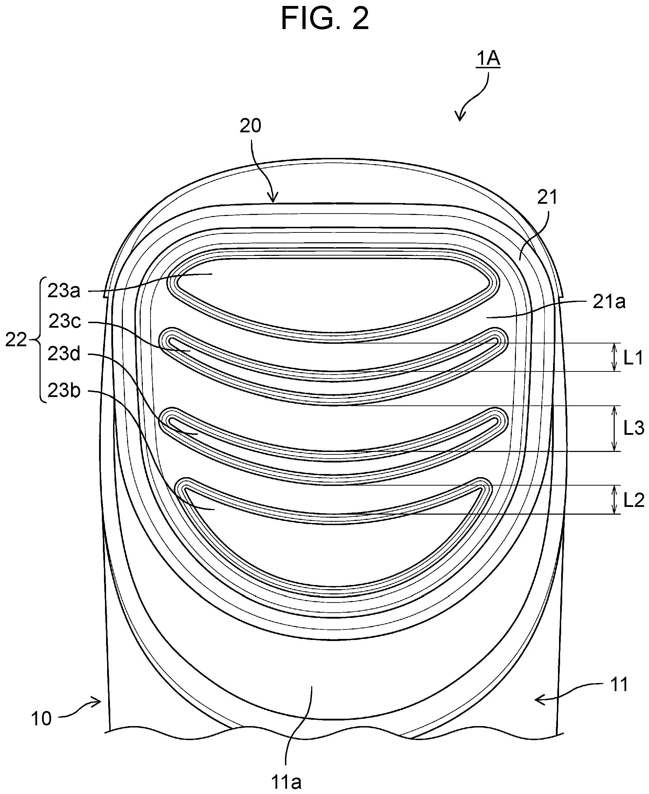

Fig. 1 is a perspective view illustrating beauty device 1A according to a first exemplary embodiment. Fig. 2 is an enlarged view of head part 20 included in beauty device 1A.

Beauty device 1A is a device that applies a beauty effect by applying a current to the skin of a user as an electric stimulus by adopting a method of performing treatment while the user holds beauty device 1A itself. Beauty device 1A includes main body 10 and head part 20.

The main body 10 includes a housing 11, a battery 12 as a power supply unit, a control board 13 on which control unit 13a is mounted, and a switch button as an operation unit. Note that control unit 13a is not limited to a dedicated hardware circuit that realizes the function, and may realize the function by a processor executing a program stored in a memory.

Housing 11 partially supports head part 20 and houses components such as battery 12 and control board 13. Housing 11 has a columnar shape with one end as top 11a and the other end as bottom 11b as a whole. At the time of treatment, the side surface part of housing 11 is gripped by the user. Hereinafter, a part of the side surface part of housing 11 and a portion facing the user side when the user grips housing 11 at the time of treatment is defined as front surface part 11c. On the other hand, a part of the side surface part of housing 11 on the opposite side of front surface part 11c is defined as back surface part 11d. Since housing 11 has such a columnar shape, when the user grips housing 11, the palm and the fingers other than the thumb of the user easily come into contact with back surface part 11d, and the thumb of the user easily comes into contact with front surface part 11c.

Top 11a supports head part 20. The surface to be top 11a is inclined with respect to a plane perpendicular to the axial direction of housing 11 such that the height on front surface part 11c side is lower than the height on back surface part 11d side. As a result, the user can easily bring head part 20 into contact with his/her skin when holding housing 11 at the time of treatment.

Battery 12 is electrically connected to control board 13. A part of control board 13 is electrically connected to electrode group 22 in head part 20.

The switch button is exposed to the outside from front surface part 11c of housing 11 and operated by the user. The switch buttons are, for example, power/mode switch 14a and level switch 14b. Power/mode switch 14a is operated when power is turned on/off, a use mode is switched, and the like. Level switch 14b is operated when the energization level is adjusted.

Head part 20 operates by being supplied with electric power, and performs treatment on the skin of the user. Head part 20 includes head main body 21 and electrode group 22.

Head main body 21 has head surface 21a facing the skin as a treatment target of the user at the time of treatment. Head main body 21 has head surface 21a on which electrode group 22 that comes into contact with the skin of the user at the time of treatment is disposed.

Electrode group 22 is, as an example, an aggregate of three or more electrodes 23 of upper electrode 23a, lower electrode 23b, and intermediate electrode. The intermediate electrode is an electrode disposed between upper electrode 23a and lower electrode 23b. In the present exemplary embodiment, electrode group 22 includes, as an example, two intermediate electrodes of first intermediate electrode 23c and second intermediate electrode 23d. That is, electrode group 22 includes, as an example, four electrodes 23 of upper electrode 23a, lower electrode 23b, first intermediate electrode 23c, and second intermediate electrode 23d. Electrode 23 is made of, for example, a titanium alloy.

Upper electrode 23a is an electrode disposed at a position farthest from bottom 11b of main body 10 on head surface 21a. Lower electrode 23b is an electrode disposed at a position closest to bottom 11b on head surface 21a. First intermediate electrode 23c is an electrode disposed at a position adjacent to upper electrode 23a on head surface 21a. Second intermediate electrode 23d is an electrode disposed at a position adjacent to lower electrode 23b on head surface 21a. That is, on head surface 21a, four electrodes 23 are arranged in parallel in a direction in which upper electrode 23a and lower electrode 23b face each other.

The planar shape of each electrode 23 included in electrode group 22 is set as follows, for example. The planar shape of upper electrode 23a may be a shape including a curved part protruding on the side facing lower electrode 23b. The planar shapes of first intermediate electrode 23c and second intermediate electrode 23d may be what is called an arcuate shape including a curved part in which side toward upper electrode 23a is concave and a curved part in which side toward lower electrode 23b is convex. The planar shape of lower electrode 23b may be a shape including a curved part that is concave on the side facing upper electrode 23a. Here, the curvatures of the curved parts included in respective electrodes 23 are set approximately equal to each other. That is, the distance between electrodes 23 adjacent to each other is approximately constant as the entire curved part. Since the planar shape of each electrode 23 is set to such a shape, each electrode 23 can efficiently flow a current along the curved surface of the skin.

Furthermore, the distance between respective electrodes 23 included in electrode group 22 is set as follows, for example. As shown in Fig. 2, the distance between upper electrode 23a and first intermediate electrode 23c is defined as first distance L1, and the distance between lower electrode 23b and second intermediate electrode 23d is defined as second distance L2. In addition, the distance between the intermediate electrodes, that is, between first intermediate electrode 23c and second intermediate electrode 23d in the present exemplary embodiment is defined as third distance L3. In this case, the respective values of first distance L1, second distance L2, and third distance L3 may be different from each other. For example, in the present exemplary embodiment, third distance L3 is longer than first distance L1 and second distance L2.

Next, an operation of beauty device 1A will be described.

First, the user operates power/mode switch 14a to turn on the power source, and then selects a use mode when performing treatment using beauty device 1A. In the present exemplary embodiment, control unit 13a can execute treatment including the following steps as a first use mode, for example.

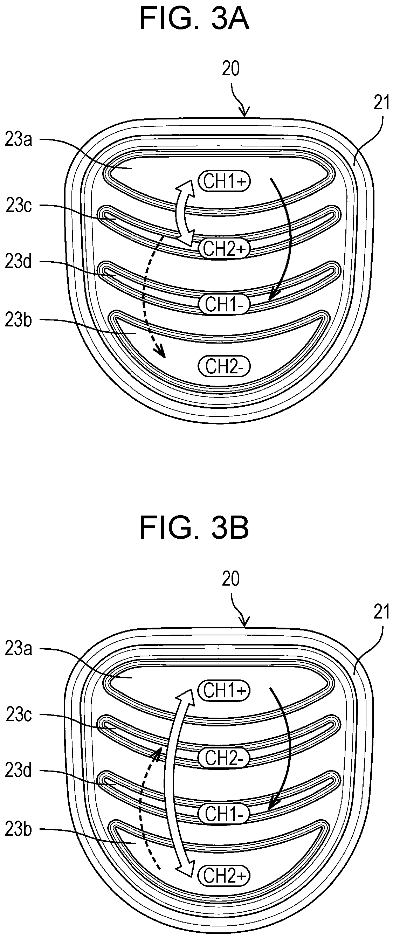

Figs. 3A to 3D are diagrams illustrating control examples of respective electrodes 23 at the time of treatment in the first use mode in time series. Figs. 3A to 3D illustrate respective electrodes 23 when viewed facing head surface 21a of head part 20.

Here, among electrodes 23 included in electrode group 22, a channel formed by a combination of electrode 23 set as the first positive electrode and electrode 23 set as the first negative electrode is defined as a "first channel". In Figs. 3A to 3D, in the first channel, "CH1+" is written on electrode 23 set as the first positive electrode, and "CH1-" is written on electrode 23 set as the first negative electrode.

Similarly, among electrodes 23 included in electrode group 22, a channel formed by a combination of electrode 23 set as the second positive electrode and electrode 23 set as the second negative electrode is defined as a "second channel". In Figs. 3A to 3D, in the second channel, "CH2+" is written on electrode 23 set as the second positive electrode, and "CH2-" is written on electrode 23 set as the second negative electrode.

In Figs. 3A to 3D, the current flow range in the skin of the user when the first channel is energized is schematically indicated by a solid arrow, and the current flow range in the skin of the user when the second channel is energized is schematically indicated by a broken arrow. Further, a range in which an interference current occurs when both the first channel and the second channel are energized is schematically indicated by a white arrow.

In the first use mode, control unit 13a executes a step of changing the setting every predetermined time while setting two or more positive electrodes and one or more negative electrodes from electrodes 23 included in electrode group 22. In the example illustrated in Figs. 3A to 3D, control unit 13a selects two positive electrodes and two negative electrodes from four electrodes 23. Control unit 13a sets the combination of the first positive electrode and the first negative electrode as a first channel, and sets the combination of the second positive electrode and the second negative electrode as a second channel. Furthermore, control unit 13a makes the characteristics of the current applied by each of the first positive electrode and the second positive electrode different from each other every predetermined time.

In addition, in the first use mode, it is assumed that a current (rectangular AC voltage) as a rectangular pulse wave that inverts polarities with each other at a constant cycle is applied to the positive pole for each set channel. At this time, the characteristic of the current that varies between the first positive electrode and the second positive electrode every predetermined time is the magnitude of the frequency. Here, the frequency of the current that can be applied by electrode 23 is assumed to be in a range of 1 Hz or more and less than 10 kHz. The difference between the frequencies of the currents of the first positive electrode and the second positive electrode is in a range of 0.1 Hz or more and less than 100 kHz. Under this condition, the frequency of the current is set to be comfortable for the user depending on the state of the skin surface of the user, the use site, or the like. In the example of the present exemplary embodiment, a current of 1000 Hz is applied from the first positive pole of the first channel, and a current of 1010 Hz is applied from the second positive pole of the second channel. On the other hand, the first negative electrode of the first channel and the second negative electrode of the second channel are set to GND.

Furthermore, in the first use mode, control unit 13a sets the first channel and the second channel such that the current flow range in the first channel intersects the current flow range in the second channel. Here, in the present exemplary embodiment, the fact that the current flow ranges in the respective channels intersect means that at least one electrode 23 selected from electrodes 23 constituting one channel is arranged between electrodes 23 constituting the other channel.

Hereinafter, steps that can be executed by control unit 13a so as to satisfy the above condition regarding the first use mode will be described in time series with reference to Figs. 3A to 3D.

Fig. 3A is a view illustrating a setting state of electrode 23 in the first stage of the first use mode. First, among four electrodes 23 included in electrode group 22, control unit 13a sets a combination of upper electrode 23a and second intermediate electrode 23d as a first channel, and sets a combination of lower electrode 23b and first intermediate electrode 23c as a second channel. This satisfies the condition that the current flow ranges in the respective channels intersect when the current is applied in the respective channels. In the first channel, control unit 13a then sets upper electrode 23a as a first positive electrode and second intermediate electrode 23d as a first negative electrode. In the second channel, the control unit 13a sets first intermediate electrode 23c as a second positive electrode and sets lower electrode 23b as a second negative electrode.

According to the setting state of electrode 23 in the first stage, first, a current of a medium frequency of 1000 Hz acts between upper electrode 23a and second intermediate electrode 23d. However, since lower electrode 23b is also set to the negative electrode, a current of a medium frequency of 1000 Hz also acts between upper electrode 23a and lower electrode 23b. On the other hand, a current of a medium frequency of 1010 Hz acts between first intermediate electrode 23c and lower electrode 23b. However, since second intermediate electrode 23d is also set to the negative electrode, a current at a medium frequency of 1010 Hz also acts between first intermediate electrode 23c and second intermediate electrode 23d. That is, a medium-frequency stimulus is given to a region corresponding to a current flow range between these electrodes in the skin of the user.

Furthermore, in the first stage, as indicated by a white arrow in Fig. 3A, a potential difference in frequency difference between the voltages output from the respective electrodes is generated between upper electrode 23a and first intermediate electrode 23c, and a low frequency current of 10 Hz acts as the interference current. At this time, first distance L1 between upper electrode 23a and first intermediate electrode 23c is shorter than the distance between upper electrode 23a and second intermediate electrode 23d. That is, the low-frequency stimulus is most felt in the region corresponding to the current flow range between upper electrode 23a and first intermediate electrode 23c in the skin of the user.

Fig. 3B is a view illustrating a setting state of electrode 23 in the second stage of the first use mode. As the control in the second stage subsequent to the first stage, control unit 13a switches the polarity of each electrode 23 in the second channel while maintaining the setting of electrode 23 in the first channel. That is, in the second channel, first intermediate electrode 23c is set as a second negative electrode, and lower electrode 23b is set as a second positive electrode.

According to the setting state of electrode 23 in the second stage, first, a current of a medium frequency of 1000 Hz acts between upper electrode 23a and second intermediate electrode 23d. However, in the second stage, since first intermediate electrode 23c is newly set to the negative electrode, a current of a medium frequency of 1000 Hz also acts between upper electrode 23a and first intermediate electrode 23c. On the other hand, a current of a medium frequency of 1010 Hz acts between lower electrode 23b and first intermediate electrode 23c. However, in the second stage, since lower electrode 23b is newly set to the positive electrode, a current of a medium frequency of 1010 Hz also acts between lower electrode 23b and second intermediate electrode 23d. That is, a medium-frequency stimulus is given to a region corresponding to a current flow range between these electrodes in the skin of the user.

Furthermore, in the second stage, as indicated by a white arrow in Fig. 3B, a potential difference in frequency difference between the voltages output from the respective electrodes is generated between upper electrode 23a and lower electrode 23b, and a low frequency current of 10 Hz acts as the interference current. That is, although a medium-frequency stimulus is given to a part of the skin of the user, a low-frequency stimulus is felt in a region corresponding to the current flow range between upper electrode 23a and lower electrode 23b, that is, the range of entire electrode group 22.

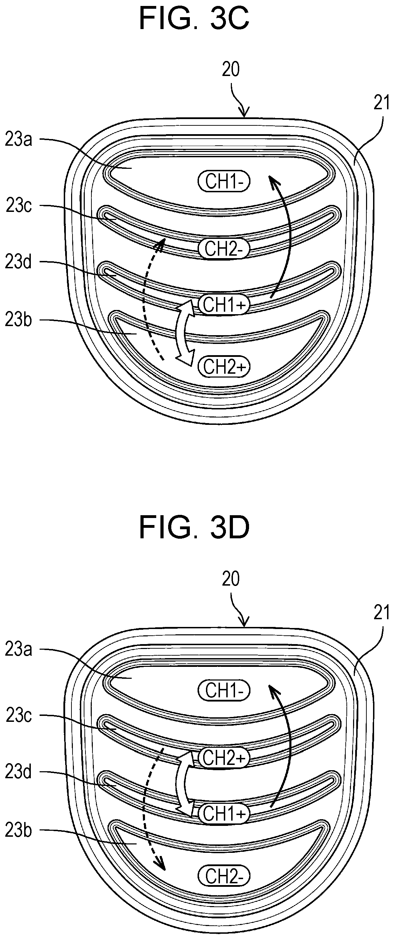

Fig. 3C is a view illustrating a setting state of electrode 23 in the third stage of the first use mode. As the control in the third stage subsequent to the second stage, control unit 13a switches the polarity of each electrode 23 in the first channel while maintaining the setting of electrode 23 in the second channel. That is, in the first channel, upper electrode 23a is set as a first negative electrode, and second intermediate electrode 23d is set as a first positive electrode.

According to the setting state of electrode 23 in the third stage, first, a current of a medium frequency of 1000 Hz acts between upper electrode 23a and second intermediate electrode 23d. However, in the third stage, since second intermediate electrode 23d is newly set to the positive electrode, a current of a medium frequency of 1000 Hz also acts between second intermediate electrode 23d and first intermediate electrode 23c. On the other hand, a current of a medium frequency of 1010 Hz acts between lower electrode 23b and first intermediate electrode 23c. However, in the second stage, since upper electrode 23a is newly set to the negative electrode, a current of a medium frequency of 1010 Hz also acts between lower electrode 23b and upper electrode 23a. That is, a medium-frequency stimulus is given to a region corresponding to a current flow range between these electrodes in the skin of the user.

Furthermore, in the third stage, as indicated by a white arrow in Fig. 3C, a potential difference in frequency difference between the voltages output from the respective electrodes is generated between second intermediate electrode 23d and lower electrode 23b, and a low frequency current of 10 Hz acts as the interference current. At this time, second distance L2 between second intermediate electrode 23d and lower electrode 23b is shorter than the distance between lower electrode 23b and first intermediate electrode 23c. That is, the low-frequency stimulus is most felt in the region corresponding to the current flow range between second intermediate electrode 23d and lower electrode 23b in the skin of the user.

Fig. 3D is a view illustrating a setting state of electrode 23 in the fourth stage of the first use mode. As the control in the fourth stage subsequent to the third stage, control unit 13a switches the polarity of each electrode 23 in the second channel while maintaining the setting of electrode 23 in the first channel. That is, in the second channel, lower electrode 23b is set as a second negative electrode, and first intermediate electrode 23c is set as a second positive electrode.

According to the setting state of electrode 23 in the fourth stage, first, a current of a medium frequency of 1000 Hz acts between upper electrode 23a and second intermediate electrode 23d. However, in the fourth stage, since lower electrode 23b is newly set to the negative electrode, a current of a medium frequency of 1000 Hz also acts between second intermediate electrode 23d and lower electrode 23b. On the other hand, a current of a medium frequency of 1010 Hz acts between lower electrode 23b and first intermediate electrode 23c. However, in the fourth stage, since first intermediate electrode 23c is newly set to the positive electrode, a current of a medium frequency of 1010 Hz also acts between first intermediate electrode 23c and upper electrode 23a. That is, a medium-frequency stimulus is given to a region corresponding to a current flow range between these electrodes in the skin of the user.

Furthermore, in the fourth stage, as indicated by a white arrow in Fig. 3D, a potential difference in frequency difference of the voltages output from the respective electrodes is generated between second intermediate electrode 23d and first intermediate electrode 23c, and a low frequency current of 10 Hz acts as the interference current. At this time, third distance L3 between first intermediate electrode 23c and second intermediate electrode 23d is shorter than any of the distance between second intermediate electrode 23d and upper electrode 23a and the distance between first intermediate electrode 23c and lower electrode 23b. That is, the low-frequency stimulus is most felt in the region corresponding to the current flow range between second intermediate electrode 23d and first intermediate electrode 23c in the skin of the user.

Then, after the control in the fourth stage is completed, control unit 13a may continuously return to the control in the first stage and repeat a series of steps from the first stage to the fourth stage.

The control unit 13a controls each electrode 23 so as to change each step from the first stage to the fourth stage at predetermined time intervals. As described above, in each step from the first to fourth stages, the stimulus by the medium frequency current and the interference stimulus by the interference current due to the difference in frequency are applied to the skin of the user. Then, the range in which the interference current is applied to the skin of the user changes for each step from the first stage to the fourth stage.

Here, the user is more likely to feel a stimulus by an interference current, which is a low frequency current, than a stimulus by a medium frequency current. On the other hand, in the first use mode, since the range in which the interference current is applied is changed every predetermined time during the treatment, a different stimulus is given to the skin of the user with time, and as a result, a high stimulus effect is easily obtained.

In the first use mode, in at least one of the steps from the first stage to the fourth stage, the magnitude or depth of the range to which the interference current is applied also changes. In the present exemplary embodiment, in the first stage and the third stage, since the values of first distance L1 and second distance L2 are equal to each other, the size or depth of the range to which the interference current is applied is also equal to each other. On the other hand, in the fourth stage, since third distance L3 is longer than first distance L1 and second distance L2, the range in which the interference current is applied is larger or deeper than in the case of the first stage and the third stage. Furthermore, in the second stage, since the interference current is applied between upper electrode 23a and lower electrode 23b, which are electrodes 23 most separated from each other, the range in which the interference current is applied is the largest or deepest in each step.

Therefore, in the second stage in which the interference current is applied between electrodes 23 that are relatively largely separated from each other, the facial expression muscle is vibrated by the electric stimulus in a deep and uniform manner in a wide range. On the other hand, in the first stage, the third stage, and the fourth stage in which the interference current is applied between electrodes 23 that are relatively close to each other, the interference current locally acts on the skin, so that a shallower stimulus can be given.

Such a first use mode is suitable, for example, as a treatment mode for promoting muscle exercise on the skin of the user. Here, the treatment for promoting muscle exercise that can be applied in the present exemplary embodiment may be electrical muscle stimulation (EMS) that excites nerves with an electric current to cause muscle contraction. According to the EMS, by using a weak current, it is possible to give a stimulus to muscles of each part of the face and obtain a tightening effect or the like.

Then, the user operates power/mode switch 14a to select the first use mode, and then brings electrode group 22 into contact with his/her skin to start the treatment. Prior to the treatment in the first use mode, the user may appropriately change the magnitude of the voltage applied from electrode 23 as the energization level by operating level switch 14b to adjust the intensity of the stimulus suitable for the user.

Although the case where the user selects the first use mode and control unit 13a performs the treatment based on the first use mode has been exemplified so far, beauty device 1A can also perform the treatment based on the following use modes other than the first use mode.

For example, in the first use mode, the frequency of the current applied from the first negative electrode of the first channel is 1000 Hz, and the frequency of the current applied from the second positive electrode of the second channel is 1010 Hz, which are constant values. On the other hand, in the second use mode replacing the first use mode, control unit 13a changes the frequency of the current applied from either the first positive electrode or the second positive electrode with time. Specifically, the frequency of the current applied from the first positive electrode is constant at 1000 Hz as in the first use mode. On the other hand, the frequency of the current applied from the second positive electrode may be changed, for example, between 1000 Hz and 1010 Hz. In this case, in the first use mode, the frequency of the interference current is constant at approximately 10 Hz, whereas in the second use mode, the frequency of the interference current fluctuates between approximately 1 Hz and 10 Hz at predetermined time intervals. Therefore, according to the second use mode, the skin of the user is given a stimulus feeling different for each time. For example, when the frequency of the interference current is about 1 Hz, a stimulus such as massaging is given to the skin of the user at intervals of one second. On the other hand, when the frequency of the interference current is about 10 Hz, a stimulus is given to the skin of the user such that tapping is performed 10 times per 1 second.

Next, effects of beauty device 1A and a method for controlling beauty device 1A will be described.

First, beauty device 1A includes electrode group 22 having three or more electrodes 23 through which current flows in the skin of the user. Further, beauty device 1A includes control unit 13a that changes the setting at predetermined time intervals while setting two or more positive electrodes and one or more negative electrodes in electrode 23 included in electrode group 22. Control unit 13a makes the characteristics of the current applied by each of the first positive electrode and the second positive electrode, which are selected from two or more positive electrodes, different from each other.

The method for controlling beauty device 1A includes a step of changing the setting at predetermined time intervals while setting two or more positive electrodes and one or more negative electrodes in electrode 23 included in electrode group 22. In this step, the characteristics of the current applied by each of the first positive electrode and the second positive electrode selected from two or more positive electrodes are different from each other.

According to beauty device 1A and the method for controlling beauty device 1A, as exemplified with reference to the series of steps from Fig. 3A to Fig. 3D, a stimulus by, for example, a medium frequency current can be given to the skin of the user between electrodes 23 according to a predetermined combination. Furthermore, not only the stimulus by the medium frequency current but also the interference stimulus by the interference current due to the difference in frequency in which the applied range changes every predetermined time can be given to the skin of the user. As a result, since beauty device 1A can automatically change the intensity of a stimulus or the range in which a stimulus is given, it is possible to give a more satisfactory stimulus to the skin of the user.

As described above, according to the present exemplary embodiment, it is possible to provide beauty device 1A and the method of controlling beauty device 1A that give a stimulus that provides a user's skin with more sense of use.

In beauty device 1A, the frequency of the current applied by electrode 23 may range from 1 Hz to less than 10 kHz.

According to beauty device 1A, for example, in the case of performing treatment on the facial skin of the user, it is possible to give a stimulus suitable for obtaining a tightening effect on each part of the facial expression muscles.

In beauty device 1A, control unit 13a may make magnitudes of the frequency of the current different from each other between the first positive electrode and the second positive electrode.

According to beauty device 1A, control unit 13a can generate the interference current to a desired magnitude or in a desired range due to the difference between the frequency of the current based on the first positive electrode and the frequency of the current based on the second positive electrode.

Further, in beauty device 1A, the difference in frequency of the current between the first positive electrode and the second positive electrode may be in a range of 0.1 Hz or more and less than 100 kHz.

According to beauty device 1A, control unit 13a can set the interference current to a low frequency current at which the stimulus applied to the skin of the user becomes relatively strong. For example, in a case where the current applied between electrodes 23 according to a predetermined combination is a medium frequency current, an interference current that is a low frequency current is clearly distinguished from the medium frequency current, and thus it can be advantageous for giving a more varied stimulus to the skin of the user.

In beauty device 1A, control unit 13a may change the frequency of the current with time at the first positive electrode or the second positive electrode.

According to this beauty device 1A, since the frequency of the interference current fluctuates every predetermined time during the treatment, it can be advantageous to give the skin of the user different stimulus feeling every time.

Further, in beauty device 1A, a first channel including a combination of a first positive electrode and a first negative electrode and a second channel including a combination of a second positive electrode and a second negative electrode are defined. In this case, control unit 13a may set the first channel and the second channel such that the current flow range in the first channel and the current flow range in the second channel intersect at least partially.

According to beauty device 1A, the interference current can be more reliably generated due to the difference between the frequency of the current based on the first positive electrode and the frequency of the current based on the second positive electrode. In addition, it is possible to widen the width of a pattern that can be set for the magnitude of the interference current or the range to which the interference current is applied.

Further, in beauty device 1A, an inter-electrode distance of two electrodes 23 selected from electrodes 23 included in electrode group 22 may be different for each selected electrode 23.

In a case where the distance between the two electrodes 23 selected from electrode group 22 is relatively short, the current energized between electrodes 23 acts locally on the skin of the user, and thus a large stimulus is likely to be obtained. On the other hand, in a case where the distance between the two electrodes 23 selected from electrode group 22 is relatively long, the current applied between electrodes 23 easily acts on a wide range of the skin of the user. Therefore, according to beauty device 1A, the stimulus to the skin of the user can be made different by making the inter-electrode distance different for each of electrodes 23.

Further, beauty device 1A may include head part 20 in which three or more electrodes 23 are arranged in parallel, and main body 10 that holds head part 20. Three or more electrodes 23 may be at least upper electrode 23a, lower electrode 23b, and a plurality of intermediate electrodes each arranged between upper electrode 23a and lower electrode 23b. Here, upper electrode 23a is electrode 23 disposed farthest from bottom 11b of main body 10. Lower electrode 23b is electrode 23 disposed closest to bottom 11b of main body 10.

According to this beauty device 1A, it is possible to give a stimulus for obtaining more sense of use to the skin of the user by adopting a method of performing treatment in a state where the user holds beauty device 1A itself.

The distance between upper electrode 23a and the intermediate electrode located closest to upper electrode 23a is defined as first distance L1. The distance between lower electrode 23b and the intermediate electrode closest to lower electrode 23b is defined as second distance L2. A distance between two intermediate electrodes adjacent to each other is defined as third distance L3. In this case, in beauty device 1A, a value of third distance L3 may be different from a value of each of first distance L1 and second distance L2.

According to beauty device 1A, in a case of adopting a method of performing treatment in a state where the user holds beauty device 1A itself, the inter-electrode distance of the electrodes can be made different for each of electrodes 23, and thus, it is possible to make the stimulus to the skin of the user different in various ways.

Here, the function of beauty device 1A can be further expanded as follows.

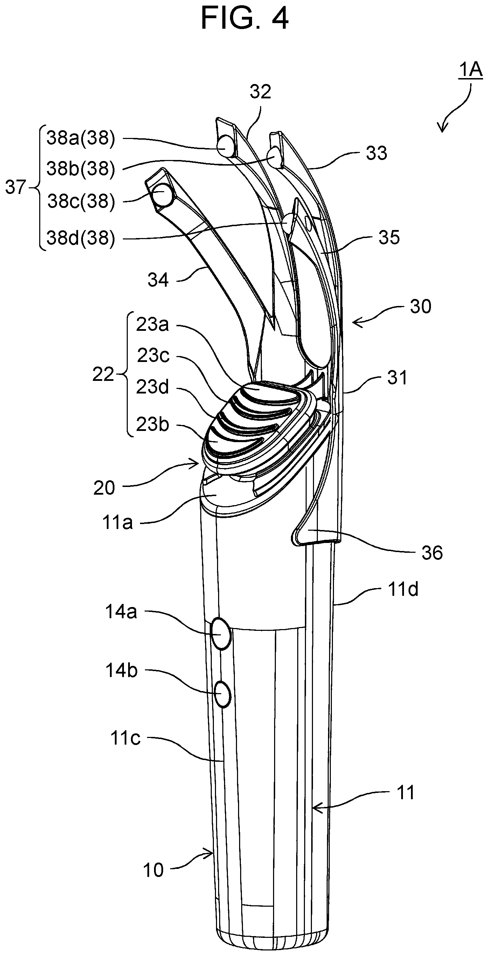

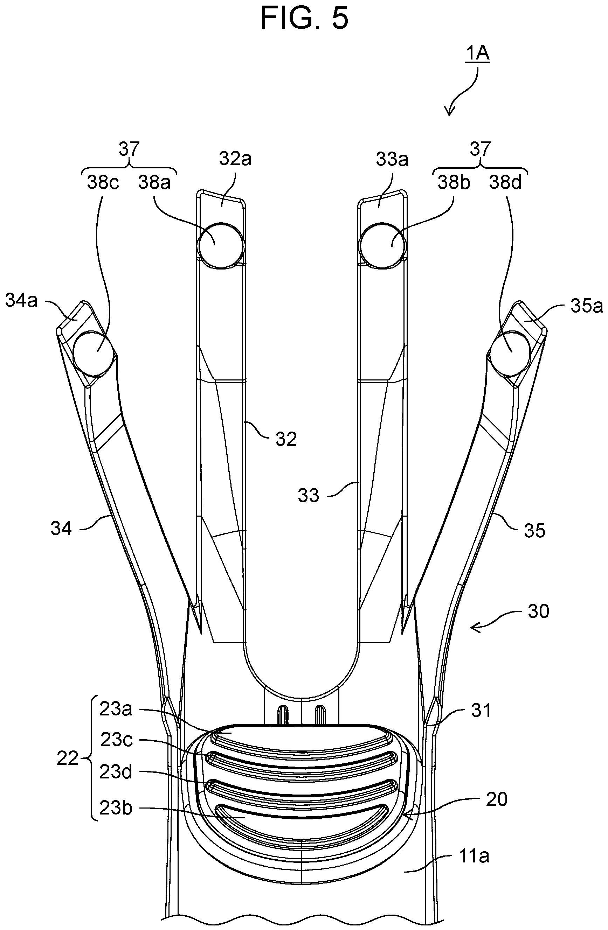

Fig. 4 is a perspective view illustrating beauty device 1A to which extended structure 30 is connected. Fig. 5 is a partially enlarged view of extended structure 30 when front surface part 11c of housing 11 in main body 10 is viewed from the same direction as when viewed from the front.

Extended structure 30 includes extended electrode group 37 that is an electrode group different from electrode group 22 included in beauty device 1A itself. Extended structure 30 has a detachable shape with respect to a distal end on top 11a side of housing 11 in beauty device 1A. Extended structure 30 includes, for example, structure main body 31, first arm 32, second arm 33, third arm 34, fourth arm 35, and connection part 36. In addition, extended structure 30 may be a resin member in which all these constituent parts are integrally molded.

Structure main body 31 supports four arms from first arm 32 to fourth arm 35 on one distal end side, and supports connection part 36 on the other distal end side.

Each of first arm 32, second arm 33, third arm 34, and fourth arm 35 holds electrode 38 at the distal end on the side opposite to the side supported by structure main body 31. First arm 32 and second arm 33 are arms extending in the same direction while being separated from each other at a constant interval. Third arm 34 is located on the side opposite to the side on which second arm 33 is located with respect to first arm 32. Specifically, third arm 34 is inclined so as to be away from first arm 32 from the side supported by structure main body 31 toward the distal end on the opposite side while the portion supported by structure main body 31 is at the same position as the portion of first arm 32. Fourth arm 35 is located on the side opposite to the side on which first arm 32 is located with respect to second arm 33. Specifically, fourth arm 35 is inclined so as to be away from second arm 33 from the side supported by structure main body 31 toward the distal end on the opposite side while the portion supported by structure main body 31 is at the same position as the portion of second arm 33. Each of first arm 32, second arm 33, third arm 34, and fourth arm 35 has a distal end bent toward the front of beauty device 1A.

In addition, first arm 32 includes first electrode 38a at first distal end 32a. Second arm 33 has second electrode 38b at second distal end 33a. Third arm 34 has third electrode 38c at third distal end 34a. Fourth arm 35 has fourth electrode 38d at fourth distal end 35a. Four electrodes 38 from first electrode 38a to fourth electrode 38d constitute extended electrode group 37, and are connected to connectors (not illustrated) provided on back surface part 11d of beauty device 1A through connection part 36 while communicating with the inside of each arm.

Connection part 36 is detachably connected to the distal end of housing 11. Connecting connection part 36 to housing 11 electrically connects control unit 13a in main body 10 and each electrode 38 included in extended electrode group 37 via connectors (not illustrated).

With such a configuration, the user can attach extended structure 30 to main body 10 of beauty device 1A to cause extended electrode group 37 included in extended structure 30 to function similarly to electrode group 22 included in head part 20.

That is, beauty device 1A includes extended electrode group 37 having three or more electrodes 38 through which current flows in the skin of the user. Further, beauty device 1A includes control unit 13a that changes the setting at predetermined time intervals while setting two or more positive electrodes and one or more negative electrodes in electrode 38 included in extended electrode group 37. Control unit 13a makes the characteristics of the current applied by each of the first positive electrode and the second positive electrode different from each other.

According to beauty device 1A using extended structure 30, the user can perform the same treatment as the treatment using electrode group 22 exemplified above using extended electrode group 37. In particular, according to the arrangement of four electrodes 38 from first electrode 38a to fourth electrode 38d provided at the distal end of each arm, beauty device 1A can be suitable for performing treatment of applying a current by bringing each electrode 38 into contact with the forehead of the user, for example, in the vicinity of the hairline of the hair.

### (Second exemplary embodiment)

In the first exemplary embodiment, the case has been exemplified where beauty device 1A adopts a method of performing treatment in a state where the user holds beauty device 1A itself. On the other hand, the beauty device according to the present disclosure may adopt a method of being worn on the face of the user.

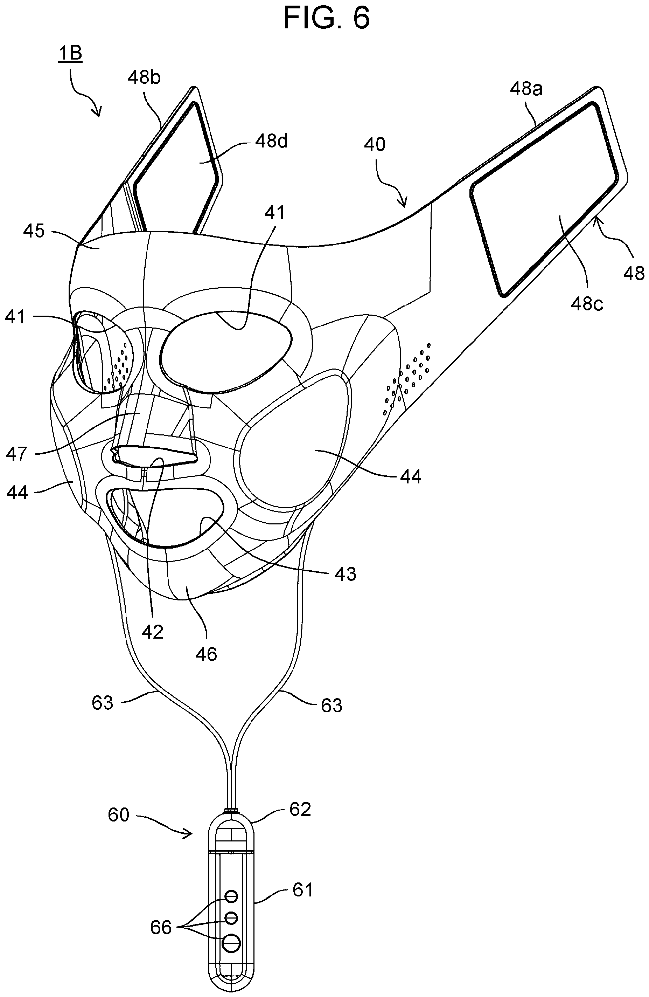

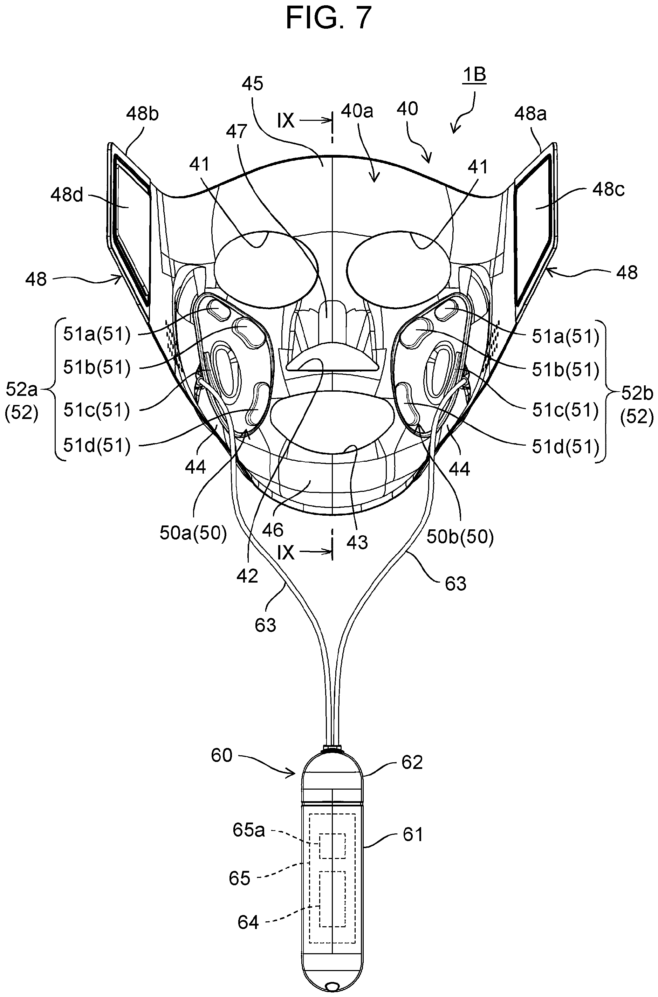

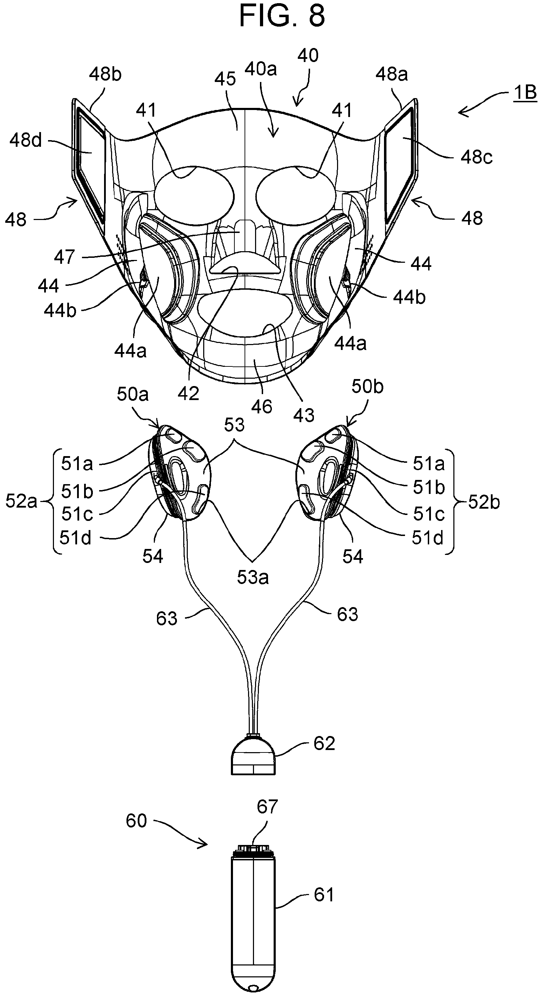

Fig. 6 is a perspective view of beauty device 1B according to the second exemplary embodiment. Fig. 7 is a plan view of beauty device 1B when mask main body 40 is viewed from a side to be worn on the face of the user. Fig. 8 is an exploded view of beauty device 1B when mask main body 40 is viewed from a side to be worn on the face of the user.

Beauty device 1B is a device that adopts a system in which at least a part of beauty device 1B is worn on the face of the user, and applies a current to the facial skin as an electric stimulus to give a cosmetic effect. Note that a beauty device adopting a method of being worn on the face of the user like beauty device 1B may be referred to as a wearable facial device, a beauty mask, or the like.

Beauty device 1B includes mask main body 40, treatment structure 50, and controller 60. Hereinafter, the upper and lower sides of mask main body 40 correspond to the upper side or the lower side in the vertical direction with reference to a state in which the user of beauty device 1B faces the straight front when the user is in a standing posture or a sitting posture. The left and right sides of mask main body 40 correspond to the left buccal side or the right buccal side of the face of the user.

Mask main body 40 is worn on the face of the user when the user uses beauty device 1B. A material of mask main body 40 is an elastic material such as silicone rubber. As a result, mask main body 40 becomes an elastic main body having flexibility as a whole, and when worn on the face, inner surface 40a is deformed following the shape of the face to be in close contact with the face. Inner surface 40a is a surface of mask main body 40 on a side facing the face in use. Furthermore, in the present exemplary embodiment, mask main body 40 has a shape that can cover almost the entire area of the face except for the eyes, the nostril, and the mouth.

First, mask main body 40 has a pair of left and right first exposure holes 41, second exposure hole 42, and third exposure hole 43 as hole parts. First exposure hole 41 is formed in the upper part of mask main body 40, and exposes the user's eyes outward when mask main body 40 is worn on the face. Second exposure hole 42 is formed below first exposure hole 41 of mask main body 40, and exposes the nostril of the user to the outside when mask main body 40 is worn on the face. Third exposure hole 43 is formed below second exposure hole 42 of mask main body 40, and exposes the user's mouth to the outside when mask main body 40 is worn on the face.

Furthermore, mask main body 40 includes cheek covering parts 44, forehead covering part 45, chin covering part 46, and nose covering part 47 as covering parts that cover the face. First, forehead covering part 45 is located above first exposure hole 41 in mask main body 40, and comes into contact with the user's forehead when mask main body 40 is worn on the face. Chin covering part 46 is located below third exposure hole 43 in mask main body 40, and contacts the chin of the user when mask main body 40 is worn on the face. nose covering part 47 is located above second exposure hole 42 so that second exposure hole 42 opens obliquely downward toward the outside in mask main body 40, and contacts the nose of the user when mask main body 40 is worn on the face. One cheek covering part 44 is located below first exposure hole 41 and on each of the left and right sides of nose covering part 47, second exposure hole 42, third exposure hole 43, and chin covering part 46, and faces one of the left and right cheeks of the user when mask main body 40 is worn on the face.

In addition, mask main body 40 has containers 44a that accommodate and hold treatment structure 50 at portions to be cheek covering parts 44 on inner surface 40a. Container 44a is provided in each of cheek covering parts 44 one on each of the left and right sides of mask main body 40. Grooves 44b communicating containers 44a may be formed in mask main body 40. Groove 44b accommodates a part of cable 63 connected to treatment structure 50.

Furthermore, mask main body 40 has belt 48 wound around the head to maintain the wearing position when being worn on the face. Belt 48 includes first belt-like body 48a connected to one of the left and right sides of mask main body 40 and second belt-like body 48b connected to the other of the left and right sides of mask main body 40. First belt-like body 48a has first hook-and-loop fastener 48c on the distal end side. On the other hand, second belt-like body 48b has second hook-and-loop fastener 48d on the distal end side. When causing mask main body 40 to be worn on the face, the user wraps first belt-like body 48a and second belt-like body 48b around the head from the side, and engages first hook-and-loop fastener 48c and second hook-and-loop fastener 48d on the back of the head. Thus, belt 48 can cause mask main body 40 to be worn on the face while applying a desired pulling force to mask main body 40.

Treatment structure 50 operates by being supplied with electric power, and performs treatment on the skin of the user. In the present exemplary embodiment, there are two treatment structures 50. One first treatment structure 50a is attached to container 44a provided in cheek covering part 44 on the left side. Other second treatment structure 50b is attached to container 44a provided in cheek covering part 44 on the right side. First treatment structure 50a and second treatment structure 50b have the same structure and are symmetrical to each other.

In addition, treatment structure 50 is detachable from mask main body 40. The user can take care of mask main body 40 and treatment structure 50 individually by detaching treatment structure 50 from container 44a. However, treatment structure 50 may be installed integrally with mask main body 40.

Treatment structure 50 includes electrode group 52 having three or more electrodes 51, silicon pad 53, and housing 54. Hereinafter, electrode group 52 included in first treatment structure 50a is referred to as first electrode group 52a, and electrode group 52 included in second treatment structure 50b is referred to as second electrode group 52b.

As an example, electrode group 52 includes four electrodes 51 of first electrode 51a, second electrode 51b, third electrode 51c, and fourth electrode 51d. Electrode 51 is made of, for example, a titanium alloy, and has a so-called button shape including a flat portion in contact with the skin of the user. In the present exemplary embodiment, the planar shape of electrode 51 is an elongated shape including a curved part partially located along the outer peripheral shape of treatment structure 50. However, the planar shape of electrode 51 is not limited to such an elongated shape, and may be other shapes such as a circular shape.

Each of electrodes 51 can be set to a positive electrode or a negative electrode, that is, GND. When at least one electrode 51 is set to the positive electrode and at least one other electrode 51 is set to the negative electrode, a potential difference is generated between these electrodes 51. Then, by energizing between these electrodes 51 in a state where electrode group 52 is in contact with the skin, an alternating current having a specific frequency is applied to the skin.

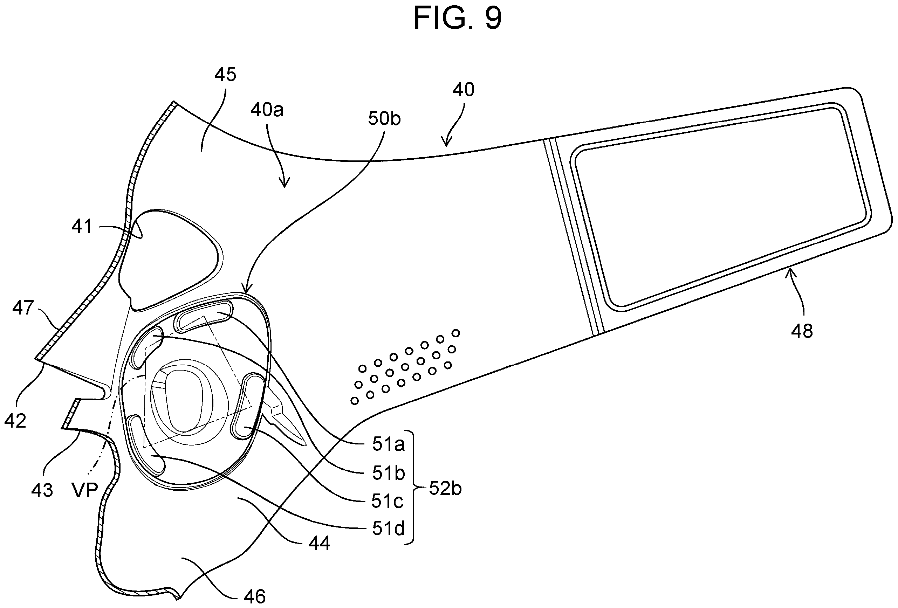

Fig. 9 corresponds to a cross section taken along line IX-IX in Fig. 7, and is a view illustrating mask main body 40 and second treatment structure 50b as viewed from the side to be worn on the face of the user.

In one treatment structure 50, the plurality of electrodes 51 are arranged so as to straddle different facial expression muscles of the user when mask main body 40 is worn on the face. Four electrodes 51 in the present exemplary embodiment are arranged so as to straddle the orbicularis oculi muscle, the zygomaticus major muscle, and the levator labii superior muscle of the user. For example, first electrode 51a may be disposed at a position extending from the eye to the vicinity of the eye corner. Second electrode 51b may be disposed near the inner corner of the eye. Third electrode 51c may be disposed near the mouth corner. In addition, fourth electrode 51d may be disposed at the back of the cheek.

In addition, in beauty device 1B, assuming a polygon VP having a centroid of a surface of each of three or more electrodes 51 as a vertex, lengths of sides of the polygon VP may be different from each other. That is, the respective values of the distance between first electrode 51a and second electrode 51b, the distance between second electrode 51b and third electrode 51c, the distance between third electrode 51c and fourth electrode 51d, and the distance between fourth electrode 51d and first electrode 51a are different from each other.

Silicon pad 53 is provided at a portion of treatment structure 50 directly facing the face when mask main body 40 is worn on the face. The surface shape of silicon pad 53 facing the face is a substantially disk shape along inner surface 40a of mask main body 40. Since silicon pad 53 has flexibility, it hardly gives discomfort to the user even when coming into contact with the face. In addition, silicon pad 53 has a plurality of through holes 53a that expose a part of electrode 51 in contact with the face of the user in a convex shape in accordance with the arrangement of each electrode 51.

Housing 54 is made of resin, for example, and holds electrode group 52 and silicon pad 53. When treatment structure 50 is attached to container 44a of mask main body 40, a part of housing 54 is engaged with a part of container 44a.

Controller 60 controls the treatment of treatment structure 50 while supplying power to electrode group 52 provided in treatment structure 50 according to the treatment of the user. Controller 60 includes, for example, controller main body 61, cap 62, and cables 63.

Controller main body 61 includes battery 64 as a power supply unit, control board 65 on which control unit 65a is mounted, and switch button 66 as an operation unit. Battery 64 is electrically connected to control board 65. Control board 65 is electrically connected to connector 67 partially exposed to the outside from controller main body 61. Switch button 66 is operated at least when switching power on/off, switching a use mode, or adjusting an energization level. Note that control unit 65a is not limited to a dedicated hardware circuit that realizes the function, and may realize the function by a processor executing a program stored in a memory.

Cap 62 is detachably attached to controller main body 61 while covering connector 67 provided on controller main body 61. When cap 62 is attached to controller main body 61, a connector (not illustrated) provided inside cap 62 is fitted to connector 67 of controller main body 61.

Cable 63 electrically connects electrode group 52 provided in treatment structure 50 and controller 60. One end of cable 63 is connected to electrode group 52, and the other end of cable 63 is connected to the connector of cap 62. Electrode group 52 is electrically connected to battery 64 and control board 65 built in controller main body 61 via cable 63.

Note that the above configuration of controller 60 is an example. For example, cable 63 may be detachable from treatment structure 50. In addition, switches for switching the power supply and the like may be provided in treatment structure 50 itself.

Next, an operation of beauty device 1B will be described.

First, the user fits the connector of cap 62 to connector 67 of controller main body 61, so that battery 64 and control board 65 on controller main body 61 side are electrically connected to respective electrodes 51 on treatment structure 50 side via cables 63. Then, when the user attaches the pair of treatment structures 50 to mask main body 40, the preparation of beauty device 1B before the treatment is completed.

Next, in a state in which inner surface 40a of mask main body 40 is in contact with the face, the user winds the first belt-like body 48a and the second belt-like body 48b around the head while pulling the first belt-like body 48a and the second belt-like body 48b from the side to the rear, and engages first hook-and-loop fastener 48c and second hook-and-loop fastener 48d on the back of the head. As a result, mask main body 40 is worn on the face while belt 48 applies a desired pulling force to mask main body 40.

Next, the user operates switch button 66 to turn on the power, and then selects a use mode for performing treatment using beauty device 1B. In the present exemplary embodiment, control unit 65a can execute treatment including the following steps for each treatment structure 50, for example, as the first use mode.

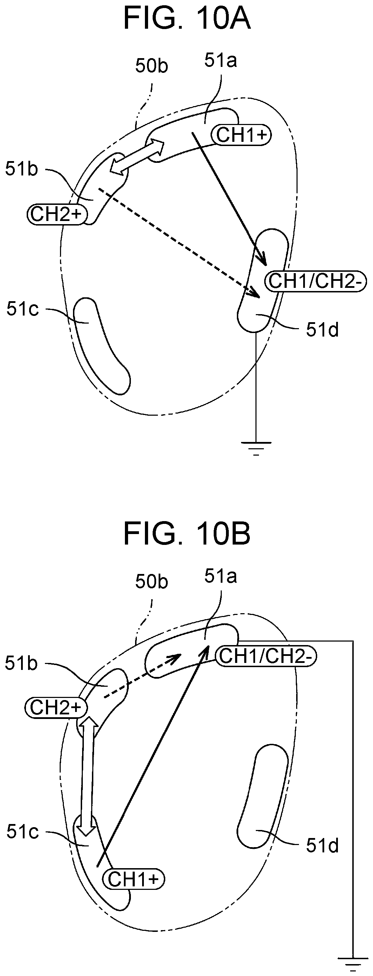

Figs. 10A to 10D are diagrams illustrating control examples of respective electrodes 51 at the time of treatment in the first use mode in time series. In Figs. 10A to 10D, as an example, the positional relationship of each electrode 51 in second treatment structure 50b illustrated in Fig. 9 is schematically illustrated.

Here, among electrodes 51 included in electrode group 52, a channel formed by a combination of electrode 51 set as the first positive electrode and electrode 51 set as the first negative electrode is defined as a "first channel". In Figs. 10A to 10D, in the first channel, "CH1+" is written on electrode 51 set as a first positive electrode, and "CH1-" is written on electrode 51 set as a first negative electrode.

Similarly, among electrodes 51 included in electrode group 52, a channel formed by a combination of electrode 51 set as the second positive electrode and electrode 51 set as the second negative electrode is defined as a "second channel". In Figs. 10A to 10D, in the second channel, "CH2+" is written on electrode 51 set as the second positive electrode, and "CH2-" is written on electrode 51 set as the second negative electrode.

In Figs. 10A to 10D, the current flow range in the skin of the user when the first channel is energized is schematically indicated by a solid arrow, and the current flow range in the skin of the user when the second channel is energized is schematically indicated by a broken arrow. Further, a range in which an interference current occurs when both the first channel and the second channel are energized is schematically indicated by a white arrow.

In the first use mode, control unit 65a executes a step of changing the setting every predetermined time while setting two or more positive electrodes and one or more negative electrodes from electrodes 51 included in electrode group 52. In the example shown in Figs. 10A to 10D, control unit 65a selects two positive electrodes and one negative electrode from four electrodes 23. Control unit 65a sets the combination of the first positive electrode and the negative electrode as a first channel, and sets the combination of the second positive electrode and the negative electrode as a second channel. That is, in the first use mode, one electrode 51 set to the negative electrode is one GND shared by the first channel and the second channel. Furthermore, control unit 65a makes the characteristics of the current applied by each of the first positive electrode and the second positive electrode different from each other electrode every predetermined time.

In addition, in the first use mode, it is assumed that a current (rectangular AC voltage) as a rectangular pulse wave that inverts polarities with each other at a constant cycle is applied to the positive pole for each set channel. At this time, the characteristic of the current that varies between the first positive electrode and the second positive electrode every predetermined time is the magnitude of the frequency. Here, the frequency of the current that can be applied by electrode 51 is assumed to be in a range of 1 Hz or more and less than 10 kHz. The difference between the frequencies of the currents of the first positive electrode and the second positive electrode is in a range of 0.1 Hz or more and less than 100 kHz. Under this condition, the frequency of the current is set to be comfortable for the user depending on the state of the skin surface of the user, the use site, or the like. In the example of the present exemplary embodiment, a current of 1000 Hz is applied from the first positive pole of the first channel, and a current of 1010 Hz is applied from the second positive pole of the second channel.

Hereinafter, steps that can be executed by control unit 65a so as to satisfy the above condition regarding the first use mode will be described in time series with reference to Figs. 10A to 10D.

Fig. 10A is a view illustrating a setting state of electrode 51 in the first stage of the first use mode. First, among four electrodes 51 included in electrode group 52, control unit 65a sets a combination of first electrode 51a and fourth electrode 51d as a first channel, and sets a combination of second electrode 51b and fourth electrode 51d as a second channel. Then, control unit 65a sets first electrode 51a of the first channel as a first positive electrode, and sets second electrode 51b of the second channel as a second positive electrode. Further, control unit 13a sets fourth electrode 51d to a negative pole shared by the first channel and the second channel.

According to the setting state of electrode 51 in the first stage, first, a current of a medium frequency of 1000 Hz acts between first electrode 51a and fourth electrode 51d. On the other hand, a current of a medium frequency of 1010 Hz acts between second electrode 51b and fourth electrode 51d. That is, a medium-frequency stimulus is given to a region corresponding to a current flow range between these electrodes in the skin of the user.

Furthermore, in the first stage, as indicated by a white arrow in Fig. 10A, a potential difference in frequency difference between the voltages output from the respective electrodes is generated between first electrode 51a and second electrode 51b, and a low frequency current of 10 Hz acts as the interference current. That is, a low-frequency stimulus is given to a region corresponding to a current flow range between first electrode 51a and second electrode 51b in the skin of the user. In this case, when a low-frequency stimulus is given, for example, muscles around the eyes of the user move.

Fig. 10B is a view illustrating a setting state of electrode 51 in the second stage of the first use mode. As the control in the second stage subsequent to the first stage, control unit 65a sets a combination of first electrode 51a and third electrode 51c as a first channel and sets a combination of first electrode 51a and second electrode 51b as a second channel among four electrodes 51 included in electrode group 52. Then, control unit 65a sets third electrode 51c of the first channel as a first positive electrode, and sets second electrode 51b of the second channel as a second positive electrode. That is, the setting of the second positive pole of the second channel in the first stage is maintained. In addition, control unit 65a sets first electrode 51a to a negative pole shared by the first channel and the second channel.

According to the setting state of electrode 51 in the second stage, first, a current of a medium frequency of 1000 Hz acts between third electrode 51c and first electrode 51a. On the other hand, a current of a medium frequency of 1010 Hz acts between second electrode 51b and first electrode 51a. That is, a medium-frequency stimulus is given to a region corresponding to a current flow range between these electrodes in the skin of the user.

Furthermore, in the second stage, as indicated by a white arrow in Fig. 10B, a potential difference in frequency difference between the voltages output from the respective electrodes is generated between second electrode 51b and third electrode 51c, and a low frequency current of 10 Hz acts as the interference current. That is, a low-frequency stimulus is given to a region corresponding to a current flow range between second electrode 51b and third electrode 51c in the skin of the user. In this case, when a low-frequency stimulus is given, for example, muscles near the lateral nasal groove, near the nasolabial fold, or under the eyes of the user move.

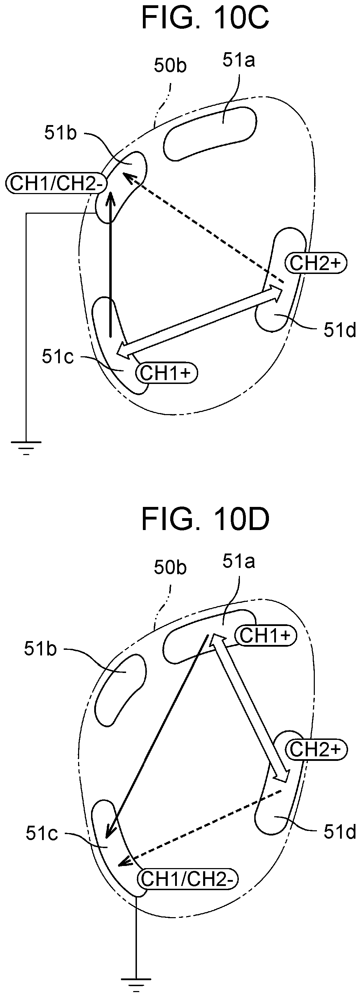

Fig. 10C is a view illustrating a setting state of electrode 51 in the third stage of the first use mode. As the control in the third stage subsequent to the second stage, control unit 65a sets the combination of third electrode 51c and second electrode 51b as a first channel and sets the combination of second electrode 51b and fourth electrode 51d as a second channel among four electrodes 51 included in electrode group 52. Then, control unit 65a sets third electrode 51c of the first channel as a first positive electrode, and sets fourth electrode 51d of the second channel as a second positive electrode. That is, the setting of the first positive pole of the first channel in the second stage is maintained. In addition, control unit 65a sets second electrode 51b to a negative pole shared by the first channel and the second channel.

According to the setting state of electrode 51 in the third stage, first, a current of a medium frequency of 1000 Hz acts between third electrode 51c and second electrode 51b. On the other hand, a current of a medium frequency of 1010 Hz acts between fourth electrode 51d and second electrode 51b. That is, a medium-frequency stimulus is given to a region corresponding to a current flow range between these electrodes in the skin of the user.

Furthermore, in the third stage, as indicated by a white arrow in Fig. 10C, a potential difference in frequency difference between the voltages output from the respective electrodes is generated between third electrode 51c and fourth electrode 51d, and a low frequency current of 10 Hz acts as the interference current. That is, a low-frequency stimulus is given to a region corresponding to a current flow range between third electrode 51c and fourth electrode 51d in the skin of the user. In this case, when a low-frequency stimulus is given, for example, cheek muscles of the user move.

Fig. 10D is a view illustrating a setting state of electrode 51 in the fourth stage of the first use mode. As the control in the fourth stage subsequent to the third stage, control unit 65a sets the combination of first electrode 51a and third electrode 51c as the first channel and sets the combination of fourth electrode 51d and third electrode 51c as the second channel among four electrodes 51 included in electrode group 52. Control unit 65a then sets first electrode 51a of the first channel as a first positive electrode, and sets fourth electrode 51d of the second channel as a second positive electrode. That is, the setting of the second positive pole of the second channel in the third stage is maintained. In addition, control unit 65a sets third electrode 51c to a negative pole shared by the first channel and the second channel.

According to the setting state of electrode 51 in the fourth stage, first, a current of a medium frequency of 1000 Hz acts between first electrode 51a and third electrode 51c. On the other hand, a current of a medium frequency of 1010 Hz acts between fourth electrode 51d and third electrode 51c. That is, a medium-frequency stimulus is given to a region corresponding to a current flow range between these electrodes in the skin of the user.

Furthermore, in the fourth stage, as indicated by a white arrow in Fig. 10D, a potential difference in frequency difference between the voltages output from the respective electrodes is generated between first electrode 51a and fourth electrode 51d, and a low frequency current of 10 Hz acts as the interference current. That is, a low-frequency stimulus is given to a region corresponding to a current flow range between first electrode 51a and fourth electrode 51d in the skin of the user. In this case, when a low-frequency stimulus is given, for example, the muscle in the vicinity of the lateral nasal groove or the cheek of the user moves.

Then, after the control in the fourth stage is completed, control unit 65a may continuously return to the control in the first stage and repeat a series of steps from the first stage to the fourth stage.

Control unit 65a controls each electrode 51 so as to change each step from the first stage to the fourth stage at predetermined time intervals. As described above, in each step from the first to fourth stages, the stimulus by the medium frequency current and the interference stimulus by the interference current due to the difference in frequency are applied to the skin of the user. Then, the range in which the interference current is applied to the skin of the user changes for each step from the first stage to the fourth stage.

In the first use mode, since the range in which the interference current is applied is changed every predetermined time during the treatment, a different stimulus is given to the skin of the user with time, and as a result, a high stimulus effect is easily obtained.

Further, in the present exemplary embodiment, regarding the polygon VP having the centroid of the surface of each of four electrodes 51 as the vertex, the lengths of the sides are different from each other. That is, in the first use mode, the size and depth of the range to which the interference current is applied also change for each step from the first stage to the fourth stage. Therefore, for example, in the third stage in which the interference current is applied between third electrode 51c and fourth electrode 51d that are relatively largely separated from each other, the facial expression muscle is uniformly vibrated by the electric stimulus in a wide range. On the other hand, in the first stage in which the interference current is applied between first electrode 51a and second electrode 51b that are relatively close to each other, the interference current locally acts on the skin, so that a larger stimulus can be given.

As in the first mode in beauty device 1A according to the first exemplary embodiment, such a first use mode may be a treatment mode for promoting muscle exercise on the skin of the user, specifically, an EMS.

Then, the user operates switch button 66 to select the first use mode as the use mode. Thus, in each of first electrode group 52a and second electrode group 52b, the treatment in the first use mode is started. Prior to the treatment in the first use mode, the user may appropriately change the magnitude of the voltage applied from electrode 51 as the energization level by operating switch button 66 to adjust the intensity of the stimulus suitable for the user.

Although the case where the user selects the first use mode and control unit 65a performs the treatment based on the first use mode has been exemplified so far, beauty device 1B can also perform the treatment based on the following use modes other than the first use mode.

For example, in the first use mode, only one negative pole shared by the first channel and the second channel is set. On the other hand, in the second use mode instead of the first use mode, only two negative poles shared by the first channel and the second channel are set.

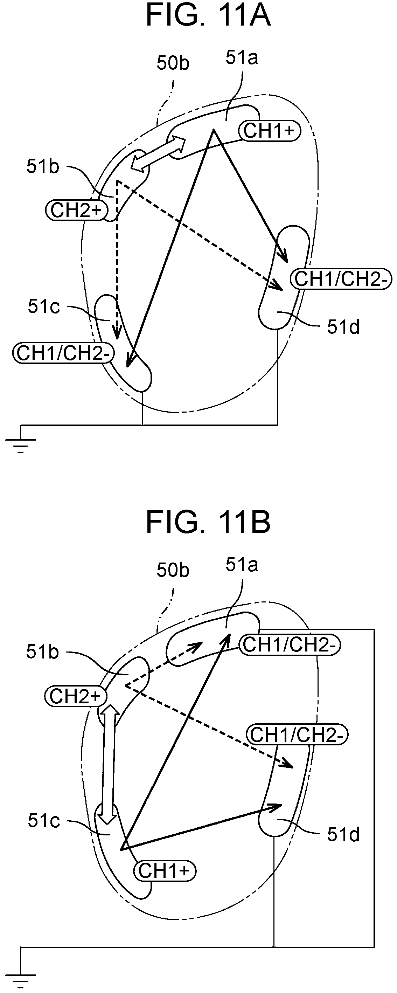

Figs. 11A to 11D are diagrams illustrating control examples of respective electrodes 51 at the time of treatment in the second use mode in time series. The drawing in Figs. 11A to 11D corresponds to the drawing in Figs. 10A to 10D regarding the first use mode.

Also in the second use mode, control unit 65a executes a step of changing the setting every predetermined time while setting two or more positive electrodes and one or more negative electrodes from electrodes 51 included in electrode group 52. In the example illustrated in Figs. 11A to 11D, control unit 65a selects two positive electrodes and two negative electrodes from four electrodes 51. Control unit 65a sets the combination of the first positive electrode and the first negative electrode as a first channel, and sets the combination of the second positive electrode and the second negative electrode as a second channel. In addition, control unit 65a makes the characteristics of the current applied by each of the first positive electrode and the second positive electrode different from each other every predetermined time. Note that conditions such as the type and frequency of the current applied from electrode 51 are the same as those in the first use mode. Furthermore, in the second use mode, control unit 65a sets the first channel and the second channel such that the current flow range in the first channel intersects the current flow range in the second channel.

Hereinafter, steps that can be executed by control unit 65a so as to satisfy the above condition regarding the second use mode will be described in time series with reference to Figs. 11A to 11D.

Fig. 11A is a view illustrating a setting state of electrode 51 in the first stage of the second use mode. First, among four electrodes 51 included in electrode group 52, control unit 65a sets a combination of first electrode 51a and third electrode 51c as a first channel, and sets a combination of second electrode 51b and fourth electrode 51d as a second channel. This satisfies the condition that the current flow ranges in the respective channels intersect when the current is applied in the respective channels. In the first channel, control unit 65a then sets first electrode 51a as a first positive electrode and third electrode 51c as a first negative electrode. In the second channel, control unit 65a sets second electrode 51b as a second positive electrode and sets fourth electrode 51d as a second negative electrode.

According to the setting state of electrode 51 in the first stage, first, a current of a medium frequency of 1000 Hz acts between first electrode 51a and third electrode 51c. However, since fourth electrode 51d is also set to the negative electrode, a current of a medium frequency of 1000 Hz also acts between first electrode 51a and fourth electrode 51d. On the other hand, a current of a medium frequency of 1010 Hz acts between second electrode 51b and fourth electrode 51d. However, since third electrode 51c is also set to the negative electrode, a current of a medium frequency of 1010 Hz also acts between second electrode 51b and third electrode 51c. That is, a medium-frequency stimulus is given to a region corresponding to a current flow range between these electrodes in the skin of the user.

Furthermore, in the first stage, as indicated by a white arrow in Fig. 11A, a potential difference in frequency difference between the voltages output from the respective electrodes is generated between first electrode 51a and second electrode 51b, and a low frequency current of 10 Hz acts as the interference current. That is, a low-frequency stimulus is given to a region corresponding to a current flow range between first electrode 51a and second electrode 51b in the skin of the user. In this case, when a low-frequency stimulus is given, for example, muscles around the eyes of the user move.

Fig. 11B is a view illustrating a setting state of electrode 51 in the second stage of the second use mode. As the control in the second stage subsequent to the first stage, control unit 65a switches the polarity of each electrode 51 in the first channel while maintaining the setting of electrode 51 in the second channel. That is, in the first channel, first electrode 51a is set as a first negative electrode, and third electrode 51c is set as a first positive electrode.

According to the setting state of electrode 51 in the second stage, first, a current of a medium frequency of 1000 Hz acts between third electrode 51c and first electrode 51a. However, in the second stage, since third electrode 51c is newly set to the positive electrode, a current of a medium frequency of 1000 Hz also acts between third electrode 51c and fourth electrode 51d. On the other hand, a current of a medium frequency of 1010 Hz acts between second electrode 51b and fourth electrode 51d. However, in the second stage, since first electrode 51a is newly set to the negative electrode, a current of a medium frequency of 1010 Hz also acts between second electrode 51b and first electrode 51a. That is, a medium-frequency stimulus is given to a region corresponding to a current flow range between these electrodes in the skin of the user.

Furthermore, in the second stage, as indicated by a white arrow in Fig. 11B, a potential difference in frequency difference between the voltages output from the respective electrodes is generated between second electrode 51b and third electrode 51c, and a low frequency current of 10 Hz acts as the interference current. That is, a low-frequency stimulus is given to a region corresponding to a current flow range between second electrode 51b and third electrode 51c in the skin of the user. In this case, when a low-frequency stimulus is given, for example, muscles near the lateral nasal groove, near the nasolabial fold, or under the eyes of the user move.

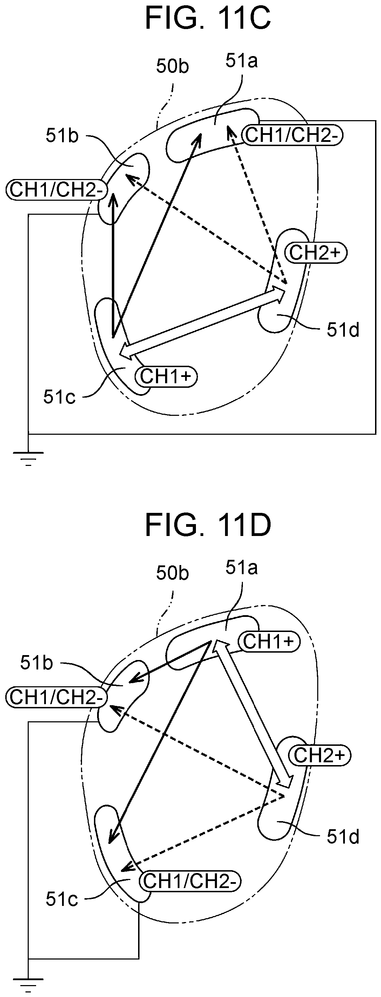

Fig. 11C is a view illustrating a setting state of electrode 51 in the third stage of the second use mode. As the control in the third stage subsequent to the second stage, control unit 65a switches the polarity of each electrode 51 in the second channel while maintaining the setting of electrode 51 in the first channel. That is, in the second channel, second electrode 51b is set as a second negative electrode, and fourth electrode 51d is set as a second positive electrode.

According to the setting state of electrode 51 in the third stage, first, a current of a medium frequency of 1000 Hz acts between third electrode 51c and first electrode 51a. However, in the third stage, since second electrode 51b is newly set to the negative electrode, a current of a medium frequency of 1000 Hz also acts between third electrode 51c and second electrode 51b. On the other hand, a current of a medium frequency of 1010 Hz acts between fourth electrode 51d and second electrode 51b. However, in the third stage, since fourth electrode 51d is newly set to the positive electrode, a current of a medium frequency of 1010 Hz also acts between fourth electrode 51d and first electrode 51a. That is, a medium-frequency stimulus is given to a region corresponding to a current flow range between these electrodes in the skin of the user.

Furthermore, in the third stage, as indicated by a white arrow in Fig. 11C, a potential difference in frequency difference between the voltages output from the respective electrodes is generated between third electrode 51c and fourth electrode 51d, and a low frequency current of 10 Hz acts as the interference current. That is, a low-frequency stimulus is given to a region corresponding to a current flow range between third electrode 51c and fourth electrode 51d in the skin of the user. In this case, when a low-frequency stimulus is given, for example, cheek muscles of the user move.

Fig. 11D is a view illustrating a setting state of electrode 51 in the fourth stage of the second use mode. As the control in the fourth stage subsequent to the third stage, control unit 65a switches the polarity of each electrode 51 in the first channel while maintaining the setting of electrode 51 in the second channel. That is, in the first channel, first electrode 51a is set as a first positive electrode, and third electrode 51c is set as a first negative electrode.

According to the setting state of electrode 51 in the fourth stage, first, a current of a medium frequency of 1000 Hz acts between first electrode 51a and third electrode 51c. However, in the fourth stage, since first electrode 51a is newly set to the positive electrode, a current of a medium frequency of 1000 Hz also acts between first electrode 51a and second electrode 51b. On the other hand, a current of a medium frequency of 1010 Hz acts between fourth electrode 51d and second electrode 51b. However, in the fourth stage, since third electrode 51c is newly set to the negative electrode, a current of a medium frequency of 1010 Hz also acts between fourth electrode 51d and third electrode 51c. That is, a medium-frequency stimulus is given to a region corresponding to a current flow range between these electrodes in the skin of the user.

Furthermore, in the fourth stage, as indicated by a white arrow in Fig. 11D, a potential difference in frequency difference between the voltages output from the respective electrodes is generated between first electrode 51a and fourth electrode 51d, and a low frequency current of 10 Hz acts as the interference current. That is, a low-frequency stimulus is given to a region corresponding to a current flow range between first electrode 51a and fourth electrode 51d in the skin of the user. In this case, when a low-frequency stimulus is given, for example, the muscle in the vicinity of the lateral nasal groove or the cheek of the user moves.

Then, after the control in the fourth stage is completed, control unit 65a may continuously return to the control in the first stage and repeat a series of steps from the first stage to the fourth stage.

Here, when the first use mode and the second use mode are compared with each other, the ranges in which the interference current occurs are the same. However, in the second use mode, since the number of negative poles, that is, the set number of GNDs is larger than that in the case of the first use mode, the stimulus by the interference current becomes weak as the number of branches of the medium frequency current becomes larger. Therefore, when the user wants to further suppress the intensity of the stimulus during the treatment, the user may select the second use mode instead of the first use mode.

Although the second use mode has been described above, similarly to the case of the first use mode, also in the second use mode, control unit 65a may change the frequency of the current applied from either the first positive electrode or the second positive electrode with time.

Next, effects of beauty device 1B and a method for controlling beauty device 1B will be described.

First, beauty device 1B includes electrode group 52 having three or more electrodes 51 through which current flows in the skin of the user. Further, beauty device 1A includes control unit 65a that changes the setting at predetermined time intervals while setting two or more positive electrodes and one or more negative electrodes in electrode 51 included in electrode group 52. Control unit 65a makes the controls of the frequencies of the current applied by each of the first positive electrode and the second positive electrode, which are selected from two or more positive electrodes, different from each other.

The method for controlling beauty device 1B includes a step of changing the setting at predetermined time intervals while setting two or more positive electrodes and one or more negative electrodes in electrode 51 included in electrode group 52. In this step, the control conditions of the frequency of the current applied by each of the first positive electrode and the second positive electrode selected from two or more positive electrodes are different from each other.

According to beauty device 1B and the method for controlling beauty device 1B, as exemplified with reference to the series of steps in Figs. 10A to 10D, a stimulus by, for example, a medium frequency current can be given to the skin of the user between electrodes 51 according to a predetermined combination. Furthermore, not only the stimulus by the medium frequency current but also the interference stimulus by the interference current due to the difference in frequency in which the applied range changes every predetermined time can be given to the skin of the user. As a result, since beauty device 1B can automatically change the intensity of a stimulus or the range in which a stimulus is given, it is possible to give a more satisfactory stimulus to the skin of the user.

As described above, according to the present exemplary embodiment, it is possible to provide beauty device 1B and a method for controlling beauty device 1B, which give a stimulus that provides a user's skin with more sense of use.

In beauty device 1B, the frequency of the current applied by electrode 51 may range from 1 Hz to less than 10 kHz.

According to beauty device 1B, it is possible to give a stimulus suitable for obtaining a tightening effect on each part of the facial expression muscles of the user.

In beauty device 1B, control unit 65a may make magnitudes of the frequency of the current different from each other between the first positive electrode and the second positive electrode.

According to beauty device 1B, control unit 65a can generate the interference current to a desired magnitude or in a desired range due to the difference between the frequency of the current based on the first positive electrode and the frequency of the current based on the second positive electrode.

Further, in beauty device 1B, the difference in frequency of the current between the first positive electrode and the second positive electrode may be in a range of 0.1 Hz or more and less than 100 kHz.

According to beauty device 1B, control unit 65a can set the interference current to a low frequency current at which the stimulus applied to the skin of the user becomes relatively strong. For example, in a case where the current applied between electrodes 51 according to a predetermined combination is a medium frequency current, an interference current that is a low frequency current is clearly distinguished from the medium frequency current, and thus it can be advantageous for giving a more varied stimulus to the skin of the user.

In beauty device 1B, control unit 65a may change the frequency of the current with time at the first positive electrode or the second positive electrode.

According to beauty device 1B, since the frequency of the interference current fluctuates every predetermined time during the treatment, it can be advantageous to a give stimulus to the skin of the user whose strength changes more delicately.

Further, in beauty device 1B, a first channel including a combination of a first positive electrode and a first negative electrode and a second channel including a combination of a second positive electrode and a second negative electrode are defined. In this case, control unit 65a may set the first channel and the second channel such that the current flow range in the first channel and the current flow range in the second channel intersect at least partially.

According to beauty device 1B, the interference current can be more reliably generated due to the difference between the frequency of the current based on the first positive electrode and the frequency of the current based on the second positive electrode. In addition, it is possible to widen the width of a pattern that can be set for the magnitude of the interference current or the range to which the interference current is applied.

Further, in beauty device 1B, an inter-electrode distance of two electrodes 51 selected from electrodes 51 included in electrode group 52 may be different for each selected electrode 51.

In a case where the distance between the two electrodes 51 selected from electrode group 52 is relatively short, the current energized between electrodes 51 acts locally on the skin of the user, and thus a large stimulus is likely to be obtained. On the other hand, in a case where the distance between the two electrodes 51 selected from electrode group 52 is relatively long, the current applied between electrodes 51 easily acts on a wide range of the skin of the user. Therefore, according to beauty device 1B, the stimulus to the skin of the user can be made different by making the inter-electrode distance different for each of electrodes 51.

Further, beauty device 1B may include mask main body 40 to be worn on the face of the user, and electrode group 52 may be provided on mask main body 40 in accordance with the positions of the left and right cheeks of the user.

According to this beauty device 1B, it is possible to give a stimulus for obtaining more sense of use to the skin of the user by adopting a method of being worn on the face of the user.

In addition, in beauty device 1B, three or more electrodes 51 may be arranged at positions across at least one muscle selected from the orbicularis oculi muscle, the zygomaticus major muscle, and the levator labii superior muscle.

According to this beauty device 1B, control unit 65a can easily select appropriate electrode 51 from the plurality of electrodes 51 so that the current flow range more matches the region on the face to which a stimulus is to be given.

In addition, in beauty device 1B, assuming a polygon VP having a centroid of a surface of each of three or more electrodes 51 as a vertex, lengths of sides of the polygon VP may be different from each other.

According to beauty device 1B, the inter-electrode distance of the electrodes is different for each of electrodes 51, and thus it is effective as a condition for variously changing the stimulus to the skin of the user particularly when adopting the system of being worn on the face of the user.

Furthermore, beauty device 1A or 1B according to each exemplary embodiment can also be defined as follows.

Beauty device 1A or the like may include an electrode group part having three or more electrodes through which currents having different characteristics flow in the skin of the user. Further, beauty device 1A and the like may include an electrode setting control unit that sets two or more positive electrodes and one or more negative electrodes for the electrodes included in the electrode group part and changes the setting every predetermined time. The electrode setting control unit may select two or more positive electrodes and perform control so as to change the characteristic of the current applied by each of the selected electrodes with time.

In beauty device 1A and the like, the characteristic of the current may be a frequency.

Further, in beauty device 1A and the like, in the electrode group part, the inter-electrode distances of the respective electrodes may be different from each other.

Further, in beauty device 1A or the like, in the electrode group part, the electrode setting control unit may perform a strong and shallow stimulus between the electrodes in which the inter-electrode distances of the respective electrodes are close to each other, and may perform a weak and deep stimulus between the electrodes in which the inter-electrode distances of the respective electrodes are in far contact with each other. That is, in beauty device 1A or the like, in the electrode group part, the electrode setting control unit may perform a strong and shallow stimulus between the electrodes in which the inter-electrode distances of the respective electrodes are close to each other than between the electrodes in which the inter-electrode distances of the respective electrodes are in far contact with each other. Further, in beauty device 1A or the like, in the electrode group part, the electrode setting control unit may perform a weak and deep stimulus between the electrodes in which the inter-electrode distances of the respective electrodes are in far contact with each other than between the electrodes in which the inter-electrode distances of the respective electrodes are close to each other.

Further, beauty device 1A or the like may be a handheld beauty device or a mask-type beauty device that can be worn on the face.

Furthermore, in beauty device 1A or the like, the current may be a low frequency current, a medium frequency current, or an interference low frequency current.

Here, the electrode group part is a component corresponding to electrode group 22 having three or more electrodes 23 or electrode group 52 having three or more electrodes 51. The electrode setting control unit is a component that can be included in control unit 13a or control unit 65a.

Note that, since the above-described exemplary embodiments are intended to exemplify the technique according to the present disclosure, various modifications, replacements, additions, and omissions can be made within the scope of the appended claims or of their equivalents.

Each of electrode group 22, extended electrode group 37, and electrode group 52 may have three or more electrodes, and electrode group 22 may have, for example, three electrodes 23 including one intermediate electrode, or may have five or more electrodes 23 including three or more intermediate electrodes. Extended electrode group 37 may include, for example, three electrodes 38 or five or more electrodes 38. Similarly, each electrode group 52 may have, for example, three electrodes 51 or five or more electrodes 51. In addition, since the number of positive electrodes may be two or more, for example, three or more positive electrodes may be provided, and the number of negative electrodes may be one or more, for example, three or more.

### INDUSTRIAL APPLICABILITY

The present disclosure is applicable to a beauty device. Specifically, for example, the present disclosure is applicable to various beauty devices for home use or business use.

### REFERENCE MARKS IN THE DRAWINGS

- 1A: beauty device
- 1B: beauty device
- 10: main body
- 11: housing
- 11a: top
- 11b: bottom
- 11c: front surface part
- 11d: back surface part
- 12: battery
- 13: control board
- 13a: control unit
- 14a: power/mode switch
- 14b: level switch
- 20: head part
- 21: head main body
- 21a: head surface
- 22: electrode group
- 23: electrode
- 23a: upper electrode
- 23b: lower electrode
- 23c: first intermediate electrode
- 23d: second intermediate electrode
- 30: extended structure
- 31: structure main body
- 32: first arm
- 32a: first distal end
- 33: second arm
- 33a: second distal end
- 34: third arm
- 34a: third distal end
- 35: fourth arm
- 35a: fourth distal end
- 36: connection part
- 37: extended electrode group
- 38: electrode
- 38a: first electrode
- 38b: second electrode
- 38c: third electrode
- 38d: fourth electrode
- 40: mask main body
- 40a: inner surface
- 41: first exposure hole
- 42: second exposure hole
- 43: third exposure hole
- 44: cheek covering part
- 44a: container
- 44b: groove
- 45: forehead covering part
- 46: chin covering part
- 47: nose covering part
- 48: belt
- 48a: first belt-like body
- 48b: second belt-like body
- 48c: first hook-and-loop fastener
- 48d: second hook-and-loop fastener
- 50: treatment structure
- 50a: first treatment structure
- 50b: second treatment structure
- 51: electrode
- 51a: first electrode
- 51b: second electrode
- 51c: third electrode
- 51d: fourth electrode
- 52: electrode group
- 52a: first electrode group
- 52b: second electrode group
- 53: silicon pad
- 53a: through hole
- 54: housing
- 60: controller
- 61: controller main body
- 62: cap
- 63: cable
- 64: battery
- 65: control board
- 65a: control unit
- 66: switch button
- 67: connector
- L1: first distance
- L2: second distance
- L3: third distance
- VP: polygon

## Claims

1. A beauty device comprising:
an electrode group including three or more electrodes through which a current flows in a skin of a user; and
a control unit that performs a setting of selecting two or more positive electrodes and one or more negative electrodes from the three or more electrodes included in the electrode group, the setting is changed at predetermined time intervals,
wherein the control unit makes characteristics of the current applied by each of a first positive electrode and a second positive electrode, which are selected from the two or more positive electrodes, different from each other.

2. The beauty device according to claim 1, wherein a frequency of the current applied by the three or more electrodes is in a range of 1 Hz or more and less than 10 kHz.

3. The beauty device according to claim 2, wherein the control unit make magnitudes of the frequency of the current different from each other between the first positive electrode and the second positive electrode.

4. The beauty device according to claim 3, wherein a difference in the frequency of the current between the first positive electrode and the second positive electrode is in a range of 0.1 Hz or more and less than 100 kHz.

5. The beauty device according to claim 2, wherein the control unit changes the frequency of the current with time at the first positive electrode or the second positive electrode.

6. The beauty device according to claim 1 or 2, wherein the control unit sets a first channel including a combination of the first positive electrode and a first negative electrode selected from the one or more negative electrodes, and a second channel including a combination of the second positive electrode and a second negative electrode selected from the one or more negative electrodes such that a flow range of the current in the first channel and a flow range of the current in the second channel intersect at least partially.

7. The beauty device according to claim 1 or 2, wherein an inter-electrode distance of two electrodes selected from the three or more electrodes included in the electrode group is different for each selected electrode.

8. The beauty device according to claim 1, further comprising:
a head part in which the three or more electrodes are arranged in parallel; and
a main body that holds the head part,
wherein the three or more electrodes include at least:
an upper electrode disposed most apart from a bottom of the main body;
a lower electrode disposed closest to the bottom of the main body; and
a plurality of intermediate electrodes each disposed between the upper electrode and the lower electrode.

9. The beauty device according to claim 8,
wherein a distance between the upper electrode and an intermediate electrode closest to the upper electrode among the plurality of intermediate electrodes is defined as a first distance,
a distance between the lower electrode and an intermediate electrode closest to the lower electrode among the plurality of intermediate electrodes is defined as a second distance, and
when a distance between two of the intermediate electrodes adjacent to each other is a third distance, a value of the third distance is different from a value of each of the first distance and the second distance.

10. The beauty device according to claim 1, further comprising a mask main body to be worn on a face of the user, wherein the electrode group is provided on the mask main body in accordance with positions of left and right cheeks of the user.

11. The beauty device according to claim 10, wherein the three or more electrodes are disposed at a position across at least one muscle selected from an orbicularis oculi muscle, a zygomaticus major muscle, and a levator labii superior muscle.

12. The beauty device according to claim 11, wherein, assuming a polygon having a centroid of a surface of each of the three or more electrodes as a vertex, lengths of sides of the polygon are different from each other.

13. A method for controlling a beauty device including an electrode group having three or more electrodes through which a current flows in a skin of a user, the method comprising a step of changing a setting at predetermined time intervals while setting two or more positive electrodes and one or more negative electrodes for the three or more electrodes included in the electrode group,
wherein, in the step, characteristics of the current applied by each of a first positive electrode and a second positive electrode selected from the two or more positive electrodes are different from each other.

14. A beauty device comprising:
an electrode group part having three or more electrodes through which currents having different characteristics flow in a skin of a user; and
an electrode setting control unit that sets two or more positive electrodes and one or more negative electrodes for the three or more electrodes included in the electrode group part and changes the setting every predetermined time,
wherein the electrode setting control unit selects the electrodes of the two or more positive electrodes, and performs control so as to change a characteristic of a current applied by each of the selected electrodes with time.

15. The beauty device according to claim 14, wherein the characteristic of the current is a frequency.

16. The beauty device according to claim 14 or 15, wherein in the electrode group part, inter-electrode distances of two electrodes selected from the three or more electrodes are different from each other.

17. The beauty device according to claim 14 or 15, wherein in the electrode group part, the electrode setting control unit performs a strong and shallow stimulus between electrodes in which inter-electrode distances of two electrodes selected from the three or more electrodes are close to each other, and performs a weak and deep stimulus between electrodes in which inter-electrode distances of two electrodes selected from the three or more electrodes are far from each other.

18. The beauty device according to claim 14 or 15, wherein the beauty device is a handheld beauty device or a mask-type beauty device wearable on a face.

19. The beauty device according to claim 14 or 15, wherein the current is a low frequency current, a medium frequency current, or an interference low frequency current.
